# FASCICULE DE BREVET EUROPEEN

(11) **EP 3 962 447 B1**
(45) Date de publication et mention de la délivrance du brevet: **08.10.2025**
(21) Numéro de dépôt: 20730314.0
(22) Date de dépôt: 04.05.2020
(51) Int. Cl.: A61K 8/9717, A61K 8/9722, A61Q 19/08

(54) **PROCÉDÉ DE SOIN COSMÉTIQUE À BASE D'EXTRAITS PHOTOACTIFS DE MICROALGUES**
KOSMETISCHE PFLEGEMETHODE AUF DER BASIS PHOTOAKTIVER MIKROALGENEXTRAKTE
COSMETIC CARE METHOD BASED ON PHOTOACTIVE EXTRACTS OF MICROALGAE

(30) Priorité: 02.05.2019 FR 1904608
(43) Date de publication de la demande: 09.03.2022
(73) Titulaire: Laboratoires d'Anjou, 75008 Paris (FR); Greensea, 63360 Saint-Beauzire (FR)
(72) Inventeur: FOUSSE, Carole, 75008 PARIS (FR); CADORET, Jean-Paul, 34140 MEZE (FR)
(74) Mandataire: Yes My Patent
(86) Numéro de dépôt international: PCT/FR2020/000159
(87) Numéro de publication internationale: WO 2020/221971

(56) Documents cités:
- FR-A1- 2 858 771
- FR-A1- 2 894 473
- FR-A1- 2 917 299
- FR-A1- 2 948 876
- FR-A1- 2 980 698
- KR-A- 20110 044 494
- US-A1- 2006 241 726
- DATABASE WPI Week 201167, Derwent World Patents Index; AN 2011-G80210

## Description

La présente invention concerne un procédé de soin cosmétique de la peau comprenant les étapes d'application sur la peau d'une composition comprenant des extraits photoactifs d'au moins une microalgue, et d'exposition à une lumière de la zone sur laquelle ladite composition a été appliquée. L'invention comprend également un dispositif lumineux pour un soin cosmétique conforme au procédé, ainsi qu'un kit comprenant une composition cosmétique comprenant des extraits photoactifs d'au moins une microalgue et un dispositif lumineux selon l'invention. L'invention comprend enfin l'utilisation du dispositif selon l'invention pour un soin anti-âge et/ou anti-rides et/ou favorisant la cicatrisation de la zone de la peau.

Le maintien d'une apparence juvénile est de nos jours un souci constant. Au cours du temps, et notamment du vieillissement chronologique et/ou photo-induit, la peau subit de nombreuses modifications et dégradations qui se traduisent, au niveau tissulaire, par une désorganisation de l'architecture de l'épiderme, de la jonction dermo-épidermique, du derme, ainsi que des systèmes d'irrigation sanguine et d'innervation, et un ralentissement ou dérèglement de différents métabolismes cellulaires, tels que ceux mis en jeu dans l'équilibre de la fonction barrière ou impliqués dans la mélanogenèse. Au niveau cellulaire, le vieillissement se traduit ainsi par une altération de la physiologie ou du métabolisme des principaux types cellulaires tels que les fibroblastes du derme, des kératinocytes de l'épiderme, et également des mélanocytes.

Le vieillissement cutané résulte de deux processus distincts et indépendants qui font intervenir des facteurs intrinsèques ou extrinsèques. Le vieillissement intrinsèque ou chrono-biologique correspond au vieillissement normal ou physiologique lié à l'âge.

Le vieillissement intrinsèque se traduit notamment par un ralentissement du renouvellement des cellules de l'épiderme et l'apparition de fines rides ou ridules. Au niveau du derme, la biosynthèse des macromolécules telles que le collagène diminue avec l'âge changeant les propriétés mécaniques du derme, d'où le relâchement cutané, un des signes cliniques du vieillissement.

Le vieillissement extrinsèque correspond au vieillissement provoqué d'une manière générale par l'environnement extérieur et correspond notamment au photo- vieillissement dû à l'exposition au soleil. Le vieillissement cutané photo-induit, c'est-à-dire provoqué suite à une exposition au soleil est encore nommé photo-vieillissement ou héliodermie. Le photo-vieillissement est le résultat, au niveau du derme, de la dégradation des fibres de collagène ayant notamment pour conséquence des altérations cliniques telles que des rides épaisses et la formation d'une peau molle et tannée. Le vieillissement de la peau est donc accéléré par une exposition chronique aux UVs. Le vieillissement extrinsèque peut également être dû au tabagisme, à une alimentation trop riche en graisse ou encore à la consommation d'alcool. Ces éléments influencent notamment le changement graduel de l'environnement redox vers un état pro-oxydant, et contribuent ainsi à un changement progressif des facteurs endothéliaux menant à la dysfonction endothéliale.

Il existe ainsi un grand nombre de soins cosmétiques dits « anti-âge » destinés à prévenir et/ou traiter le vieillissement, en particulier le photo-vieillissement et leurs signes, notamment à prévenir l'apparition des rides et à réduire de façon visible les rides déjà présentes, mais aussi atténuer ou supprimer les tâches de sénescence (dues à l'âge). Parmi ces soins cosmétiques on peut citer, par exemple, l'application régulière de lotions ou de crèmes dites « anti-âge » ou « anti-rides », comprenant différents actifs cosmétiques.

De manière analogue au vieillissement, notamment à la suite d'une blessure ou d'une chirurgie, la peau est fragilisée et l'organisme entame le processus de cicatrisation, permettant la reconstitution des tissus. Ce processus fait appel à de nombreux types cellulaires et permet d'arrêter l'hémorragie, de protéger l'organisme, d'assainir les tissus à reconstruire, et finalement de refermer la plaie.

Un nombre important de soins cosmétiques pro-cicatrisants sont disponibles aujourd'hui, principalement sous forme de crème ou de lotion, et comprennent différents actifs cosmétiques.

Parmi ces actifs cosmétiques anti-âge, anti-rides et/ou cicatrisants, les microalgues apparaissent comme des sources privilégiées en lipides, protéines, polysaccharides, vitamines (B1, B6, B12, C, E, K..), pigments et antioxydants, lesdits pigments ayant également un rôle d'antioxydants. On compte parmi ceux-ci des caroténoïdes, des phycobiliprotéines (phycoérythrine et phycocyanine), la phycocyanine, la phycoérythrine, l'astaxanthine ou encore le Beta-carotène.

Ainsi, de nombreuses compositions cosmétiques utilisent des extraits de microalgues en temps qu'anti-âge et anti-rides. On peut notamment citer la demande de brevet CN 104224608, décrivant une émulsion anti-âge et anti-rides comprenant des extraits de spiruline, la demande de brevet US 2005/0220810 décrivant une composition anti-âge et anti-rides comprenant des extraits de chlorelle, ou encore la demande JP 2007176832 décrivant une composition anti-âge comprenant des extraits de Aphanizomenon.

De manière similaire, on peut citer le brevet US 7285266, décrivant une composition cosmétique comprenant des extraits de Thalassiosira fluviatilis ou Thalassiosira weissflogii.

Ces extraits de microalgues permettent notamment, parmi tous leurs bienfaits, grâce à leurs propriétés antioxydantes de réduire le stress oxydatif des cellules de l'épiderme.

A l'inverse des effets anti-oxydants traditionnellement recherchées par l'Homme du métier dans les microalgues comme principe actif cosmétique, la Demanderesse a développé des compositions cosmétiques anti-âge, anti-rides, et/ou favorisant la cicatrisation de la peau comprenant des microalgues, dont l'activité oxydante a été significativement augmentée grâce au couplage d'une application de lumière, permettant l'activation de production de dérivés réactif de l'oxygène (ROS).

En effet, plutôt que de capitaliser sur les propriétés naturelles antioxydantes des microalgues, la Demanderesse a mis au point un procédé permettant d'activer les microalgues à l'aide de la lumière, qui vont ainsi augmenter leur production de dérivés réactif de l'oxygène (ROS) et augmenter l'état d'oxydation des cellules de l'épiderme, ainsi qu'entraîner une augmentation dose dépendante du monoxyde d'azote permettant une augmentation de la synthèse du collagène et une diminution de la libération des cytokines pro-inflammatoires et des mettalo-proteinases. La Demanderesse a pu observer de manière surprenante que l'activité cellulaire ainsi stimulée va permettre une restauration de la peau, des ridules et une augmentation de son élasticité, et dans le même temps, la lumière active le processus naturel de fabrication des différents constituants de la peau, et notamment la stimulation de l'activité des fibroblastes, entrainant l'augmentation de la production de collagène et de fibres, et donc un effet anti-age, anti-rides, et favorisant la cicatrisation, la régénération cellulaire et la vascularisation.

Un premier objet de l'invention est un procédé de soin cosmétique d'une zone de la peau comprenant les étapes successives de :
- Application sur la zone de la peau d'une composition cosmétique comprenant des extraits photoactifs d'au moins une microalgue choisie parmi : *Dunaliella salina, Dunaliella tertiolecta, Tetraselmis suecica, Chlamydomonas reinhardtii, Haematococus pluvialis, Tetraselmis tetrathele, Rhodomonas salina, et*/*ou Rhodella maculata* ;
- Exposition de la zone de la peau sur laquelle ladite composition a été appliquée à une source lumineuse émettant une lumière rouge à une longueur d'onde comprise entre 625 et 670 nm, de préférence 645 nm, et d'une intensité de 140 à 180 µmol.s-1.m-2 photons, de préférence 155 à 170 µmol.s-1.m-2 photons, le temps d'exposition à la lumière durant de 30 secondes à 40 minutes.

Par microalgue est entendu selon l'invention un organisme photoautotrophe chlorophylliens unicellulaire. On compte les microalgues eucaryotes, caractérisées par une paroi cellulaire et un noyau, comprenant notamment les chlorophytes, les chrysophytes, les pyrrophytes, les coccolithophytes, les diatomées, les euglénophytes, les rhodophytes, et les trebouxiophytes, et les procaryotes, également nommées « cyanobactéries », ne possédant pas de noyau et de paroi cellulaire, et comprenant les cyanophytes.

De manière préférée selon l'invention, ladite au moins une microalgue est photoactive.

Par extraits ou microalgues photoactif(ve)s sont entendus selon l'invention des extraits ou microalgues qui par exposition à la lumière, de préférence rouge, déclenchent une réaction d'oxydation avec une production de ROS supérieure d'au moins 50% à la production sans exposition à la lumière desdits extraits, de préférence une production de ROS supérieur de 100% , ainsi qu'une augmentation dose dépendante du monoxyde d'azote permettant une augmentation de la synthèse du collagène et une diminution de la libération des cytokines pro-inflammatoires et des mettalo-proteinases. Le terme photoréactif peut alternativement être utilisé au terme photoactif selon la présente invention.

Par source lumineuse est entendue un dispositif émettant de la lumière.

Tetraselmis suecica est une microalgue verte halophile, appartenant à la classe des Chlorodendrophytes.

Dunaliella salina est une microalgue verte halophile, appartenant à la classe des Chlorophytes, qui se développe spontanément et préférentiellement dans les milieux lagunaires très salés.

Rhodomonas salina, anciennement Pyrenomonas salina, est une algue de la famille des cryptophytes, retrouvée dans tous les milieux aquatiques, est largement utilisé comme aliment pour les cultures de copépodes.

Rhodella maculata ou Rhodella violacea est une algue rouge unicellulaire de la famille des Rhodellaceae.

Haematochococcus pluvialis est une algue verte unicellulaire d'eau douce de la famille des Haematococcaceae.

Porphyridium cruentum est une espèce d'algue rouge de la famille des Porphyridiophycées.

Cyanophora paradoxa est une algue de la famille des Glaucocystaceae.

Cylindrotheca closterium est une diatomée marine vivant dans les environnements intertidaux.

Diacronema lutheri est une haptophycée utilisée dans l'aquaculture comme nourriture pour les invertébrés marins et particulièrement pour les bivalves.

Selenastrum capricornutum ou Raphidocelis subcapitata est une microalgue utilisée communément comme bioindicateur du niveau de nutriments de substance toxique dans l'eau douce, car elle est très sensible à la présence de substances toxiques telles que les métaux.

Synechococus sp. est une espèce de cyanobactéries unicellulaires vivant majoritairement en milieu marin.

Isochrysis sp. est une algue unicellulaire comprenant une grande quantité de fucoxanthine.

Tisochrysis lutea est une haptophycée très utilisée comme aliment en aquaculture.

Phaedodactylum tricornutum est une espèce de diatomée marine cultivée notamment comme nourriture pour les mollusques et les poissons.

Arthrospira platensis est une espèce de de la famille des Phormidiaceae.

Stichococcus bacillaris est une algue terrestre retrouvée notamment sur le sol, les murs de pierre, les tuiles, les troncs d'arbres, le verre, les berges humides.

Xanthonema sp. est une espèce d'algue brune.

Nostoc sp., est une cyanobactérie de la famille des Nostocaceae.

Dunaliella Tertiolecta est une microalgue verte capable dans certaines circonstances d'accumuler des lipides.

Pseudanabaena galeata est une bactérie photosynthétique de la classe de cyanophycées

Chaetoceros calcitrans est une espèce de diatomée de la famille des Chaetocerotaceae.

Chlamydomonas reinhardtii, souvent surnommée levure verte, est une espèce d'algue verte.

Tetraselmis tetrathele est une algue verte contenant une quantité importante d'antioxydants.

De manière particulièrement préférée selon l'invention, les extraits de microalgues sont des extraits de Dunaliella Salina.

De manière particulièrement préférée selon l'invention, les extraits de microalgues sont des extraits de Tetraselmis Suecica.

De manière particulièrement préférée selon l'invention, les extraits de microalgues sont des extraits de Dunaliella Salina et Tetraselmis Suecica.

De manière préférée selon l'invention, les extraits sont des extraits liquides de microalgues obtenus de préférence après culture des microalgues, centrifugation et broyage du culot. Ces extraits peuvent être hydrosolubles et/ou liposolubles.

De manière encore plus préférée selon l'invention, ces extraits sont hydrosolubles.

De manière préférée selon l'invention, ladite composition cosmétique comprenant des extraits de Dunaliella Salina comprend :
- Entre 0,0001 % et 80% en poids de la composition d'extraits de Dunaliella Salina, de préférence entre 0,0001 et 50 % en poids de la composition.
- QSP 100 d'eau
- 2,50% en poids de la composition de glycérine
- 0,40% en poids de la composition de triethanolamine
- 15% en poids de la composition d'alcool
- 1% en poids de la composition de coco caprylate caprate
- 1% en poids de la composition d'isopropyl miristate
- 0,1% en poids de la composition de parfum

De manière préférée selon l'invention, ladite composition cosmétique comprenant des extraits de Tetraselmis Suecica comprend :
- Entre 0,0001 % et 78 % en poids de la composition d'extraits de Tetraselmis Suecica, de préférence entre 0,0001 et 50 % en poids de la composition.
- QSP 100 d'eau
- 7,00% en poids de la composition d'huile d'amande douce
- 4,50% en poids de la composition de C12-15 alkyl benzoate
- 5,65% en poids de la composition de steareth
- 1,90% en poids de la composition de glyceryl stearate
- 1,40% en poids de la composition de dimethicone
- 0,25% en poids de la composition de carbomer
- 0,25% en poids de la composition de sodium benzoate
- 0,20% en poids de la composition de parfum
- 0,18% en poids de la composition de sodium benzoate
- 0,10% en poids de la composition d'EDTA

De manière préférée selon l'invention, ladite composition cosmétique comprenant des extraits de Dunaliella Salina et de Tetraselmis Suecica comprend :
- Entre 0,0001% et 76% en poids de la composition d'extraits de Tetraselmis Suecica, de préférence entre 0,0001 et 50 % en poids de la composition.
- Entre 0,0001% et 76% en poids de la composition d'extraits de Dunaliella Salina, de préférence entre 0,0001 et 50 % en poids de la composition.
- QSP 100 d'eau
- 4,00% en poids de la composition d'huile de coton
- 3,2% en poids de la composition de glycerine
- 4,00% en poids de la composition d'huile de tournesol
- 3,00% en poids de la composition d'octyldodecanol
- 3,70% en poids de la composition de propanediol
- 1,5% en poids de la composition de sodium acrylates copolymer
- 1,10% en poids de la composition de caprylic/capric triglyceride
- 0,8% en poids de la composition de pentylene glycol
- 0,8% en poids de la composition d'aluminum starch octenylsuccinate
- 0,70% en poids de la composition de benzyl alcohol
- 0,50% en poids de la composition de lecithin
- 0,20% en poids de la composition de coco-caprylate/caprate
- 0,20% en poids de la composition de tocopherol
- 0,08% en poids de la composition d'EDTA
- 0,06% en poids de la composition de dehydroacetic acid

De manière préférée selon l'invention, ladite lumière rouge est une lumière rouge d'une longueur d'onde de 645 nm, et d'une intensité de 155 à 170 µmol.s⁻¹.m⁻² photons.

De manière préférée selon l'invention, l'exposition à la lumière est réalisée à l'aide de diodes électroluminescentes, de laser et/ou de lumière intense pulsée.

Par lumière intense pulsée (Intense Pulse Light ou IPL, en anglais) aussi appelée lampe flash est entendue selon l'invention une technologie comprenant des lampes polychromatiques à décharge de haute intensité remplies d'un gaz rare, le xénon, par exemple. Elles fonctionnent en convertissant l'énergie électrique stockée en salves intenses d'énergie rayonnante. La lumière émise couvre les régions du spectre de la lumière de l'ultraviolet, du visible et de l'infrarouge, c'est-à-dire de 350 nm à 1200 nm environ, dont la lumière rouge d'une longueur d'onde de 625 à 670 nm, de préférence 645 nm.

Par diode électroluminescente est entendu selon l'invention des composants électroniques qui, lorsqu'ils sont traversés par un courant électrique émettent de la lumière basse fréquence. Elle émet une lumière que l'on considère comme froide car elle ne produit pas de chaleur. Les diodes électroluminescentes émettent une lumière dans un spectre qui s'étend du visible à l'infrarouge.

De manière préférée selon l'invention, ladite diode électroluminescente émet une lumière rouge d'une longueur d'onde de 625 à 670 nm, de préférence 645 nm.

De manière préférée selon l'invention, le temps d'exposition à la lumière est compris de 1 secondes à 60 minutes, de préférence de 30 secondes à 40 minutes, de manière encore plus préférée de 10 à 20 minutes.

Par temps d'exposition à la lumière est entendu selon l'invention le temps durant lequel la peau de l'individu est exposée à la lumière.

De manière préférée selon l'invention la source lumineuse est placée à une distance de 2 mm à 10 cm de la zone de la peau à traiter.

De manière préférée selon l'invention, la source lumineuse éclaire à une intensité entre 3 et 150 J/cm².

Selon un second aspect, l'invention concerne un dispositif lumineux pour un soin cosmétique conforme au procédé selon l'invention, ledit dispositif consistant en un boitier comprenant une source lumineuse, ladite source lumineuse émettant une lumière dans un spectre de longueurs d'onde compris entre 625 et 670 nm, et d'une intensité de 140 à 180 µmol.s⁻¹.m⁻² photons, de préférence entre 155 et 170 µmol.s⁻¹.m⁻² photons.

De manière préférée selon l'invention, ladite source lumineuse émet une lumière rouge à une longueur d'onde de 645 nm.

De manière préférée selon l'invention, ladite source lumineuse comprend au moins une LED émettant une lumière rouge dans un spectre de longueurs d'onde compris entre 625 et 670 nm, et d'une intensité de de 140 à 180 µmol.s-1.m-2 photons, de préférence entre 155 et 170 µmol.s-1.m-2 photons.

De manière préférée selon l'invention, ladite au moins une LED émet une lumière rouge à une longueur d'onde de 645 nm.

De manière préférée selon l'invention, la source lumineuse représente une surface de 0,15 cm² à 2900 cm².

Selon un mode de réalisation de l'invention, la source lumineuse représente de préférence une surface de 0,25 à 1450 cm².

De manière préférée selon l'invention, ledit dispositif prend la forme d'un masque, d'un panneau ou d'un appareil manuel.

Par masque est entendu selon l'invention un dispositif capable d'exposer à de la lumière tout ou une partie d'un visage. La source lumineuse pourra représenter une surface d'environ la surface d'un visage soit environ 500cm2.

Par appareil à usage manuel est entendu selon l'invention un dispositif tenant dans la main et permettant d'exposer la peau d'un individu de manière localisée. Un exemple d'appareil manuel peut être un stylo, la source lumineuse pourra alors représenter une surface d'environ 0,15 à 2 cm2 ou un dispositif de la taille d'un rasoir électrique, la source lumineuse pourra alors représenter une surface d'environ 2 à 7 cm2.

Par panneau est entendu selon l'invention un dispositif capable d'exposer une très grande partie du corps d'un individu à de la lumière. Ce type de dispositif peut même aller jusqu'à exposer le corps entier d'un individu. La source lumineuse pourra alors représenter une surface de 550 cm2 à plus de 1500 cm2.

Selon un troisième aspect, l'invention concerne un Kit comprenant :
- Une composition cosmétique comprenant des extraits photoactifs d'au moins une microalgue choisie parmi : *Dunaliella Salina, Dunaliella tertiolecta, Tetraselmis suecica, Chlamydomonas reinhardtii, Haematococus pluvialis, Tetraselmis tetrathele, Rhodomonas salina, et*/*ou Rhodella maculata* ;
- Un dispositif lumineux pour un soin cosmétique selon l'invention.

De manière préférée selon l'invention, ladite composition comprend des extraits de Tetraselmis suecica.

De manière préférée selon l'invention, ladite composition comprend des extraits des extraits de Dunaliella salina.

De manière préférée selon l'invention, ladite lumière est une lumière rouge d'une longueur d'onde comprise entre 625 et 670 nm, de préférence 645 nm.

Selon un quatrième aspect, l'invention concerne l'utilisation du dispositif selon l'invention pour un soin anti-âge et/ou anti-rides et/ou favorisant la cicatrisation de la zone de la peau.

### FIGURES

[Fig. 1] : Production de ROS suite à une exposition lumineuse (645nm) de 25 minutes (en gris) pour plusieurs extraits photoactifs de micro-algues à un facteur de dilution de 500. En noir sont exposés les résultats quand les extraits sont gardés à l'obscurité.
[Fig. 2] : Influence de la durée d'exposition sur la production de molécules oxydatives (ROS) en fonction des extraits. Les extraits provenant de Dunaliella Salina (DS) (Fig. 2A), de Tetraselmis suecica (TS) (Fig. 2B) et de Rhodomonas salina (RS) (Fig. 2C), sont laissés à l'obscurité (i), ou exposées 1 minute (ii), 10 minutes (iii) ou en continue (iv) à une exposition lumineuse à 645nm.
[Fig. 3] : Gènes évalués durant l'étude de l'efficacité sur l'expression des ARNm de cellules de la peau par RT-qPCR microfluidique.
[Fig. 4] : Module LEDs rouges (longueur d'onde = 645nm) préréglé à 160µmol/m2/s utilisé pour la photoactivation (Exemple 5).

### EXEMPLES

### Exemple 1 : Préparation des échantillons

Caractérisation de la production de molécules oxydatives chez les extraits de micro-algues :
Les extraits des algues Dunaliella Salina (DS), Dunaliella tertiolecta (TD), Tetraselmis suecica (TS), Chlamydomonas reinhardtii (CR), Haematococus pluvialis (HP), Tetraselmis tetrathele (TT), Rhodomonas salina (RS), Porphyridium purpureum (PP), Rhodella maculata (RM) sont sélectionnées pour caractériser l'augmentation de la production de molécules oxydatives quand elles sont stimulées à la lumière.

Pour ce faire, les algues sont mises en cultures de 200 mL. Après comptage cellulaire à une longueur d'onde de 680 nm, 100 à 150 mL de la solution contenant les algues sont ensuite centrifugés 10 minutes à 12000 rpm.

Le culot est ensuite broyé, et le broyat est ajusté à 5.0.10⁵ cellules/mL dans 150 mL de PBS.

Après comptage cellulaire à une longueur d'onde de 680 nm, ce broyat ajusté est ensuite centrifugé pendant 10 minutes à 12000 rpm.

Enfin, une filtration à 0,2µm du surnageant est réalisée pour obtenir l'échantillon prêt à l'analyse.

Les échantillons constitués d'extraits de ces algues une fois prêts à l'analyse sont mis soit :
1. A l'obscurité, et 15µL de milieu réactionnel sont ajoutés à 30 mL de surnageant filtré à 0.2 µm ;
2. A l'obscurité, et 15µL de milieu réactionnel sont ajoutés à 30 mL de surnageant filtré à 0.2 µm, suivi d'une exposition lumineuse continue à 645nm d'une intensité de 166 µmol.s-1.m-2
3. A l'obscurité, et 30 mL de surnageant filtrés à 0.2 µm sont exposés à un bullage avec du diazote pendant 30 minutes et ensuite mélangés avec 15µL de milieu réactionnel. Une étape d'exposition lumineuse est ensuite effectuée, à 645nm d'une intensité de 166 µmol.s-1.m-2

### Exemple 2 : Screening des souches

Les différents extraits sont ensuite étudiés en les gardant à l'obscurité ou exposés pendant 25 minutes à une exposition lumineuse de 645 nm, à un facteur de dilution de 500, par des analyses de fluorescences effectuées à l'aide d'un spectrofluorométre. Les paramètres sont les suivants :
Ex:500 Em:522
Ex.scanning range 495∼505
Em.scanning range 400∼650

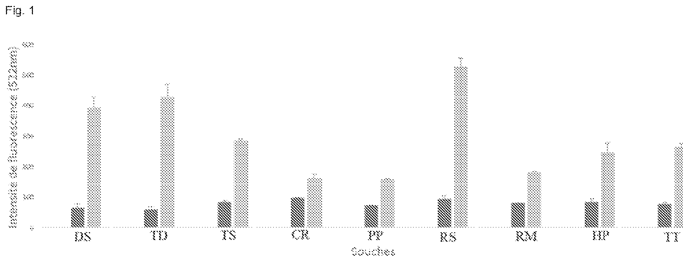

Les résultats sont exposés en figure 1.

Ces résultats montrent une augmentation de la production de ROS après une exposition lumineuse pour tous les extraits étudiés.

De manière détaillée, la production de ROS est 5 fois supérieure pour les extraits de DS, de 5.5 fois supérieure pour les extraits de TD, de 3.6 fois supérieure pour les extraits de TS, de 2.1 fois pour les extraits de CR, de 2 fois pour les extraits de PP, de 6.8 fois pour les extraits de RS, de 2.3 fois pour les extraits de RM, de 3.1 fois pour les extraits de HP et de 3.4 fois pour les extraits de TT.

### Exemple 3 : Influence de la durée d'exposition :

Les extraits sont ensuite étudiés en fonction de la durée d'exposition. Les 4 stades étudiés sont :
- Extraits gardés à l'obscurité
- Extraits 1 minute à une exposition lumineuse à 645nm
- Extraits 10 minutes à une exposition lumineuse à 645nm
- Extraits exposés en continue à une exposition lumineuse à 645nm

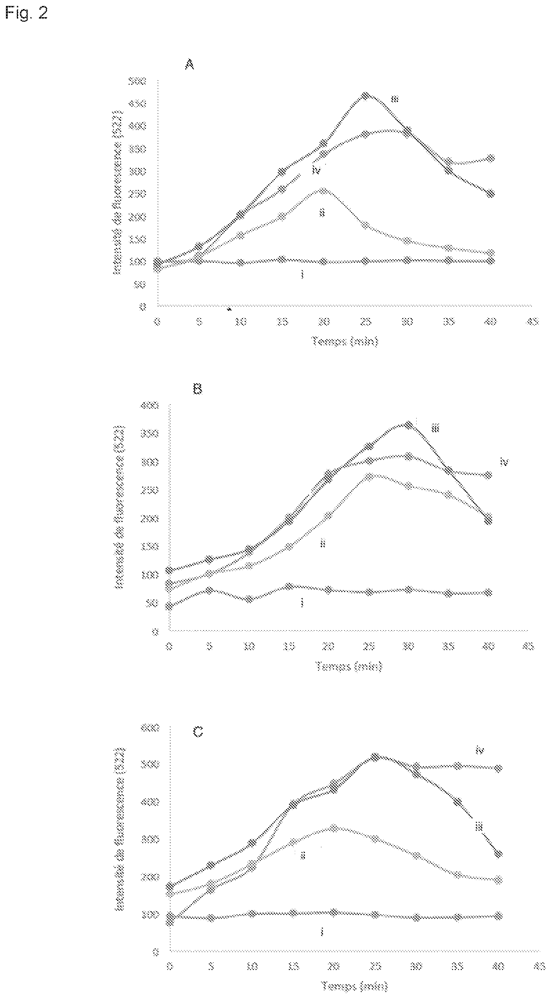

Les résultats pour DS (Fig.2A), TS (Fig.2B) et RS (Fig.2C) sont exposés en figure 2.

Ces résultats montrent une production de ROS constante à l'obscurité, quelle que soit l'extrait de micro-algue.

Pour l'exposition à 1 minute, les extraits de DS, TS et RS montrent une augmentation de la production de ROS qui s'atténue après environ 20/25 minutes. La production est croissante pour atteindre un maximum à 20/25 minutes, et redescend ensuite. On observe ainsi au maximum une augmentation d'environ 2.5 fois pour DS, de 5 fois pour TS, et d'environ 3 fois pour RS.

Après une exposition de 10 minutes, les extraits de DS, TS et RS montrent une augmentation de la production de ROS supérieure à celle caractérisée après une exposition à 1 minute. La production est croissante pour atteindre un maximum à 25/30 minutes, et redescend ensuite. On observe ainsi au maximum une augmentation d'environ 4.5 fois pour DS, de 7 fois pour TS, et de 5 fois pour RS, par rapport à l'absence d'exposition.

A exposition en continue, on remarque que les courbes sont proches de celles après une exposition de 10 minutes. La production est ainsi croissante pour obtenir un maximum aux alentours de 25/30 minutes, et redescend ensuite. Pour DS, on observe une production maximale après 25 minutes, de 3.5 fois supérieure à celle à l'obscurité, étant donc légèrement inférieure à l'exposition de 10 minutes. Durant les 25 premières minutes, l'augmentation progressive de la production de ROS est similaire à celle caractérisée pour une exposition à 10 minutes. Cependant, après 35 minutes, l'exposition en continue semble montrer une production supérieure de ROS à celle après une exposition à 10 minutes. Pour TS, on observe une production maximale après 30 minutes, de 6 fois supérieure à celle à l'obscurité, étant donc légèrement inférieure à l'exposition de 10 minutes. Durant les 25 premières minutes, l'augmentation progressive de la production de ROS est similaire à celle caractérisée pour une exposition à 10 minutes. Cependant, après 35 minutes, l'exposition en continue semble montrer une production supérieure de ROS à celle après une exposition à 10 minutes. Concernant RS, on observe une production maximale après 25 minutes, de 5 fois supérieure à celle à l'obscurité, étant donc identique à l'exposition de 10 minutes. Cependant, après 35 minutes, l'exposition en continue semble montrer une production durable supérieure de ROS à celle après une exposition à 10 minutes.

En conclusion, il n'y a pas de croissance continue de la réponse, traduite par l'atteinte d'un plateau spécifique à chaque souche de la production maximale de ROS, obtenu aux alentours de 25/30 minutes et pour un taux d'exposition de 10 minutes. De plus, on voit bien que l'arrêt de la production de ROS est conditionné par la durée de l'exposition, les expositions à 1 et 10 minutes montrant une diminution de la production de ROS après 20 à 30 minutes.

### Exemple 4 : Evaluation clinique sur 30 sujets d'un soin cosmétique anti-âge à l'aide d'un dispositif émettant de la lumière à la longueur d'onde de 645 nm

L'étude clinique porte au total sur 30 femmes âgées de 35 à 70 ans.

### a) Réalisation de la séance d'application de la composition cosmétique et application de lumière à 645 nm

Un examen clinique dermatologique de la tonicité de la peau (analyse au toucher) et des rides ou ridules est fait (scores semi-quantitatifs). Des macrophotographies (sujet non maquillé, poses standards) sont prises. Des mesures de la surface des rides, de leur profondeur et de la rugosité de la peau sont réalisées à J0 et J28 avec un appareil de mesure (Skin Station).

Application de 0,4g de composition sur le visage.

Exposition de la zone de la peau de la patte d'oie à dispositif lumineux émettant une lumière à une longueur d'onde comprise entre comprise entre 625 et 670 nm.

### Application de la source lumineuse à 645 nm (durée, distance, intensité)

### c) Conclusion

On observe une amélioration clinique après soin par la composition et le dispositif chez 27 des 30 sujets.

La tolérance du produit est excellente, et l'appréciation cosmétologique globalement très bonne.

Le procédé de soin cosmétique selon l'invention permet ainsi de diminuer les rides de la peau de façon importante.

Exemple 5 : Évaluation de l'efficacité sur l'expression des ARNm de cellules de la peau par RT-qPCR microfluidique post-activation des extraits par la lumière.

L'ensemble de ces manipulations est réalisé dans la pénombre.

### Matériel :

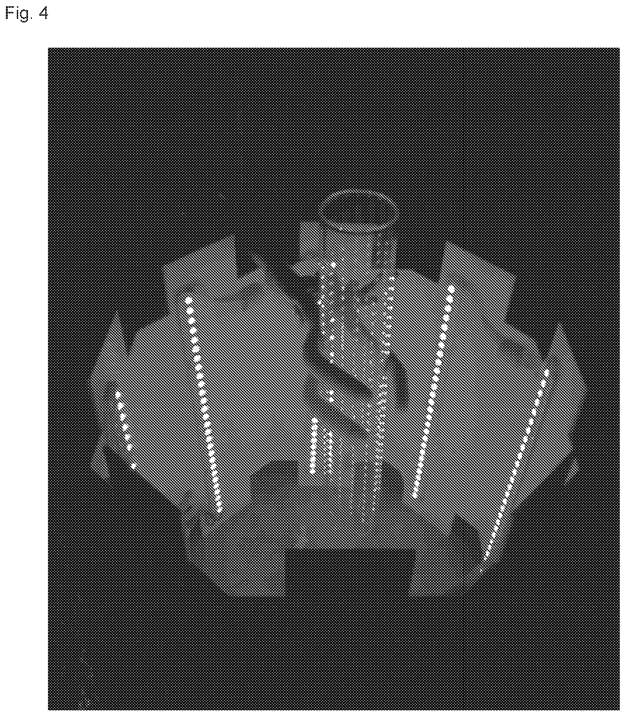

- Module LEDs rouges (longueur d'onde = 645nm) préréglé à 160µmol/m2/s , illustré en figure 4.
- Tube à essai en verre de 20mL
- Pince de serrage
- Support avec tige

### Protocole :

Dans la pénombre 5mL d'extrait sont versés dans le tube à essai.

Le tube a essai est ensuite placé au centre du module de LEDs en le fixant sur la tige du support grâce à la pince de serrage.

Le module de LEDs rouges est ensuite allumé, et la lumière est laissée agir pendant 10 minutes.

A l'issue de ces 10 minutes, la lumière est coupée et l'échantillon reste dans la pénombre.

L'extrait contenu dans le tube est prélevé à l'aide d'une pipette, sans agiter.

L'extrait photoactivé est utilisé extemporanément dans la suite du protocole.

L'expérimentation est menée en triplicata (n=3) sur des kératinocytes épidermiques humains normaux (NHEK) et des fibroblastes dermiques humains normaux (NHDF), où les extraits activés tel que ci-dessus sont utilisés.

### Méthode d'analyse :

Les ARNm sont extraits et reverse-transcrits en ADNc. L'expression des gènes est quantifiée par qPCR et normalisée par l'expression des gènes de référence. Afin de confirmer un effet activateur ou inhibiteur, les valeurs sont comparées à la condition de référence après mise en contact avec les extraits.

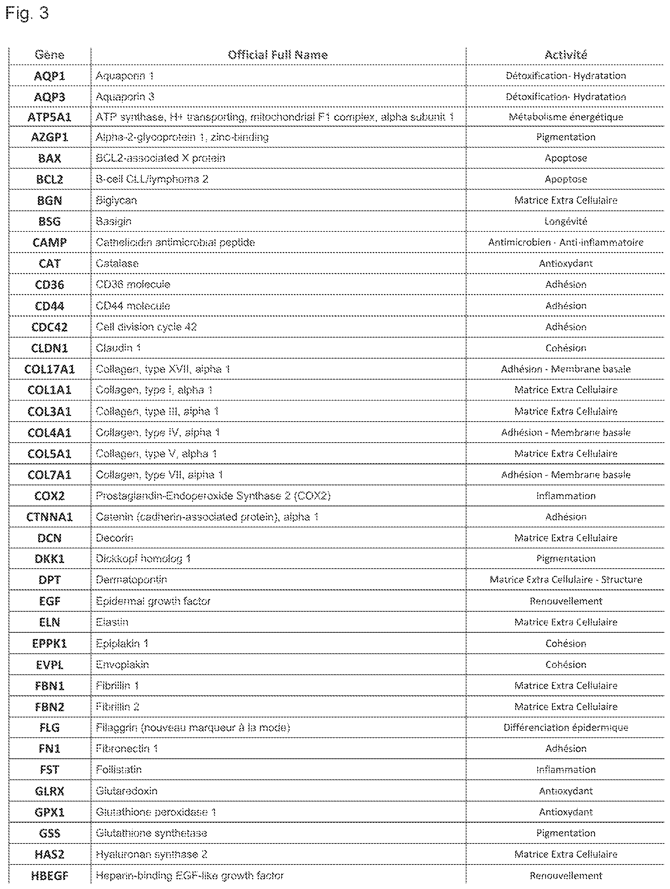

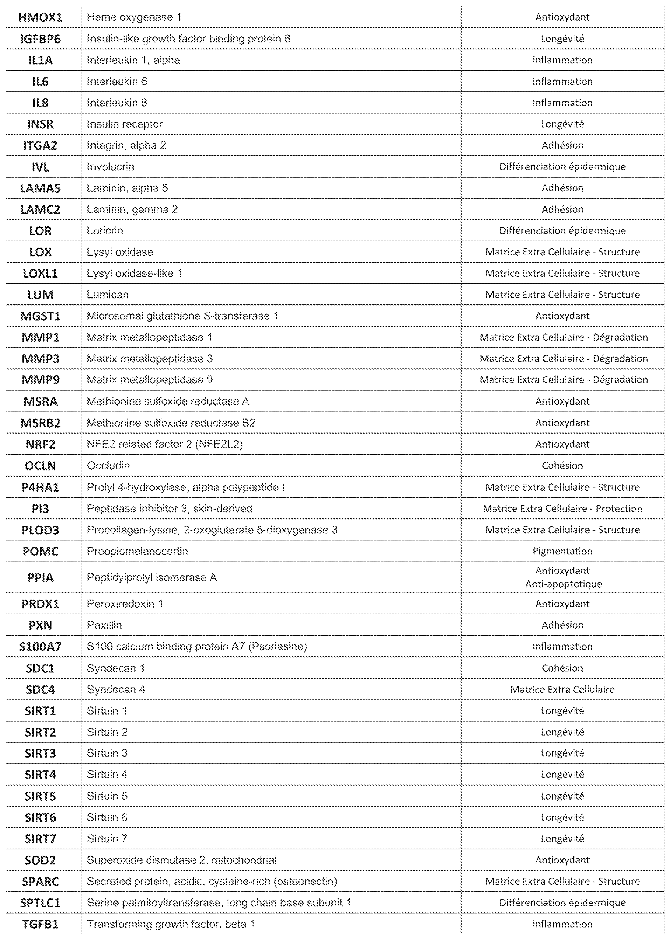

Les gènes évalués sont indiqués en Figure 3.

Une augmentation de l'expression des gènes Claudine 1, Collagène, Fibrilline 1 et 2, est observée, ainsi que de manière générale des gènes antioxydants. Dans le même temps est également observée une diminution de l'expression des gènes des métallopeptidase, 1, 3 et 9, et de l'inflammation.

En conclusion, il est observé une diminution de l'expression des gènes favorisant le vieillissement cutané.

## Revendications

1. Procédé de soin cosmétique d'une zone de la peau comprenant les étapes successives de :
- Application sur la zone de la peau d'une composition cosmétique comprenant des extraits photoactifs d'au moins une microalgue choisie parmi : *Dunaliella salina, Dunaliella tertiolecta, Tetraselmis suecica, Chlamydomonas reinhardtii, Haematococus pluvialis, Tetraselmis tetrathele, Rhodomonas salina, et*/*ou Rhodella maculata* ;
- Exposition de la zone de la peau sur laquelle ladite composition a été appliquée à une source lumineuse émettant une lumière rouge à une longueur d'onde comprise entre 625 et 670 nm, de préférence 645 nm, et d'une intensité de 140 à 180 µmol.s-1.m-2 photons, de préférence 155 à 170 µmol.s-1.m-2 photons, le temps d'exposition à la lumière durant de 30 secondes à 40 minutes.

2. Procédé de soin cosmétique selon la revendication précédente, **caractérisé en ce que** les extraits photoactifs de microalgues sont des extraits de *Dunaliella salina.*

3. Procédé de soin cosmétique selon la revendication 1, **caractérisé en ce que** les extraits photoactifs de microalgues sont des extraits de *Tetraselmis suecica.*

4. Procédé de soin cosmétique selon la revendication 1, **caractérisé en ce que** les extraits photoactifs de microalgues sont des extraits de *Rhodomonas salina.*

5. Procédé de soin cosmétique selon la revendication 1, **caractérisé en ce que** les extraits de microalgues sont des extraits de Dunaliella Salina et Tetraselmis Suecica.

6. Procédé de soin cosmétique selon l'une quelconque des revendications précédentes, **caractérisé en ce que** l'exposition à la lumière rouge est réalisée à l'aide de diodes électroluminescentes, de laser et/ou de lumière intense pulsée.

7. Dispositif lumineux pour un soin cosmétique conforme au procédé selon l'une quelconque des revendications précédentes, ledit dispositif consistant en un boitier comprenant une source lumineuse, **caractérisé en ce que** ladite source lumineuse émet une lumière rouge dans un spectre de longueurs d'onde compris entre 625 et 670 nm, et d'une intensité de 140 à 180 µmol.s⁻¹.m⁻² photons, de préférence entre 155 et 170 µmol.s⁻¹.m⁻² photons.

8. Utilisation du dispositif selon la revendication 7 pour un soin anti-âge et/ou anti-rides et/ou favorisant la cicatrisation de la zone de la peau.

9. Kit pour le soin de la peau comprenant :
- Une composition cosmétique comprenant des extraits photoactifs d'au moins une microalgue choisie parmi : *Dunaliella Salina, Dunaliella tertiolecta, Tetraselmis suecica, Chlamydomonas reinhardtii, Haematococus pluvialis, Tetraselmis tetrathele, Rhodomonas salina, et*/*ou Rhodella maculata* ;
- Un dispositif lumineux pour un soin cosmétique selon la revendication 7.

10. Kit pour le soin de la peau selon la revendication 9, **caractérisé en ce que** ladite composition comprend des extraits photoactifs de Tetraselmis suecica et/ou de Dunaliella salina.

## Patentansprüche

1. Verfahren für kosmetische Pflege eines Hautbereichs, umfassend die aufeinanderfolgenden Schritte:
- Auftragen einer kosmetischen Zusammensetzung auf den Hautbereich, umfassend photoaktive Extrakte von mindestens einer Mikroalge, die ausgewählt ist aus: *Dunaliella salina, Dunaliella tertiolecta, Tetraselmis suecica, Chlamydomonas reinhardtii, Haematococus pluvialis, Tetraselmis tetrathele, Rhodomonas salina und*/*oder Rhodella maculata;*
- Aussetzen des Hautbereichs, auf den die Zusammensetzung aufgetragen wurde, gegenüber einer Lichtquelle, die rotes Licht mit einer Wellenlänge zwischen 625 und 670 nm, vorzugsweise 645 nm, und mit einer Intensität von 140 bis 180 µmol.s-1.m-2 Photonen, vorzugsweise 155 bis 170 µmol.s-1.m-2 Photonen, aussendet, wobei die Dauer des Aussetzens gegenüber dem Licht von 30 Sekunden bis 40 Minuten beträgt.

2. Verfahren für kosmetische Pflege nach dem vorstehenden Anspruch, **dadurch gekennzeichnet, dass** es sich bei den photoaktiven Mikroalgenextrakten um Extrakte von *Dunaliella salina* handelt.

3. Verfahren für kosmetische Pflege nach Anspruch 1,
**dadurch gekennzeichnet, dass** es sich bei den photoaktiven Mikroalgenextrakten um Extrakte von *Tetraselmis suecica* handelt.

4. Verfahren für kosmetische Pflege nach Anspruch 1,
**dadurch gekennzeichnet, dass** es sich bei den photoaktiven Mikroalgenextrakten um Extrakte von *Rhodomonas salina* handelt.

5. Verfahren für kosmetische Pflege nach Anspruch 1,
**dadurch gekennzeichnet, dass** es sich bei den photoaktiven Mikroalgenextrakten um Extrakte von Dunaliella Salina und Tetraselmis Suecica handelt.

6. Verfahren für kosmetische Pflege nach einem der vorstehenden Ansprüche, **dadurch gekennzeichnet, dass** das Aussetzen gegenüber Rotlicht mittels Leuchtdioden, eines Lasers und/oder intensivem gepulstem Licht durchgeführt wird.

7. Leuchtvorrichtung für kosmetische Pflege gemäß dem Verfahren nach einem der vorstehenden Ansprüche, wobei die Vorrichtung aus einem Gehäuse besteht, umfassend eine Lichtquelle, **dadurch gekennzeichnet, dass** die Lichtquelle Rotlicht in einem Wellenlängenspektrum zwischen 625 und 670 nm und mit einer Intensität von 140 bis 180 µmol.s⁻¹.m⁻² Photonen, vorzugsweise zwischen 155 und 170 µmol.s⁻¹.m⁻² Photonen, aussendet.

8. Verwendung der Vorrichtung nach Anspruch 7 für eine Anti-Aging- und/oder Antifalten-Pflege und/oder ein Fördern der Heilung des Hautbereichs.

9. Hautpflegeset, umfassend:
- eine kosmetische Zusammensetzung, umfassend photoaktive Extrakte von mindestens einer Mikroalge, die ausgewählt ist aus: *Dunaliella Salina, Dunaliella tertiolecta, Tetraselmis suecica, Chlamydomonas reinhardtii, Haematococus pluvialis, Tetraselmis tetrathele, Rhodomonas salina und*/*oder Rhodella maculata;*
- eine Lichtvorrichtung für eine kosmetischen Pflege nach Anspruch 7.

10. Hautpflegeset nach Anspruch 9, **dadurch gekennzeichnet, dass** die Zusammensetzung photoaktive Extrakte von Tetraselmis suecica und/oder Dunaliella salina umfasst.

## Claims

1. Method for cosmetically caring for a region of the skin comprising the successive steps of:
- applying, to the skin region, a cosmetic composition comprising photoactive extracts of at least one microalgae chosen from: *Dunaliella salina, Dunaliella tertiolecta, Tetraselmis suecica, Chlamydomonas reinhardtii, Haematococus pluvialis, Tetraselmis tetrathele, Rhodomonas salina, and*/*or Rhodella maculata;*
- exposing the skin region to which said composition has been applied to a light source emitting red light at a wavelength of between 625 and 670 nm, preferably 645 nm, and with an intensity of 140 to 180 µmol.s-1.m-2 photons, preferably 155 to 170 µmol.s-1.m-2 photons, the light exposure time lasting from 30 seconds to 40 minutes.

2. Cosmetic care method according to the preceding claim,
**characterized in that** the photoactive extracts of microalgae are *Dunaliella salina* extracts.

3. Cosmetic care method according to claim 1, **characterized in that** the photoactive extracts of microalgae are *Tetraselmis suecica* extracts.

4. Cosmetic care method according to claim 1, **characterized in that** the photoactive microalgae extracts are *Rhodomonas salina* extracts.

5. Cosmetic care method according to claim 1, **characterized in that** the microalgae extracts are Dunaliella Salina and Tetraselmis Suecica extracts.

6. Cosmetic care method according to any of the preceding claims, **characterized in that** exposure to red light is carried out using light-emitting diodes, laser and/or intense pulsed light.

7. Lighting device for cosmetic care in accordance with the method according to any of the preceding claims, said device consisting of a housing comprising a light source, **characterized in that** said light source emits red light in a wavelength spectrum between 625 and 670 nm, and with an intensity of 140 to 180 µmol.s⁻¹.m⁻² photons, preferably between 155 and 170 µmol.s⁻¹.m⁻² photons.

8. Use of the device according to claim 7 for anti-aging and/or anti-wrinkle care and/or promoting healing of the skin region.

9. Skin care kit comprising:
- a cosmetic composition comprising photoactive extracts of at least one microalgae chosen from: *Dunaliella Salina, Dunaliella tertiolecta, Tetraselmis suecica, Chlamydomonas reinhardtii, Haematococus pluvialis, Tetraselmis tetrathele, Rhodomonas salina, and*/*or Rhodella maculata;*
- a lighting device for cosmetic care according to claim 7.

10. Skin care kit according to claim 9, **characterized in that** said composition comprises photoactive extracts of Tetraselmis suecica and/or Dunaliella salina.
